# EUROPÄISCHE PATENTSCHRIFT

(11) **EP 0 512 262 B1**
(45) Veröffentlichungstag und Bekanntmachung des Hinweises auf die Patenterteilung: **05.07.1995**
(21) Anmeldenummer: 92105904.4
(22) Anmeldetag: 06.04.1992
(51) Int. Cl.: A61B 17/16, A61B 17/56, A61B 19/02

(54) **Schraubenlader**
Screw loader
Chargeur à vis

(30) Priorität: 10.05.1991 CH 1405/91
(43) Veröffentlichungstag der Anmeldung: 11.11.1992
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- EP-A- 0 059 044
- US-A- 2 835 377
- US-A- 3 171 408
- US-A- 4 441 492
- US-A- 4 444 180

## Beschreibung

Die Erfindung bezieht sich auf eine Vorrichtung gemäss dem Oberbegriff des Anspruchs 1.

Bei der operativen Frakturversorgung im maxillofacialen Bereich, sowie im Hand- und Fuss-Bereich, kann eindeutig die Tendenz festgestellt werden, dass immer kleinere Implantate bevorzugt werden. Der Grund dafür ist das allgemein grösser werdende Verständnis über die biomechanischen Grundlagen der Osteosynthese. Im Bereich der maxillofacialen Frakturbehandlung kann Dank der Miniaturisierung Von Implantaten mehr Rücksicht auf das kosmetische Resultat einer Osteosynthese genommen werden.

Im Bereich der Handchirurgie können Bewegungseinschränkungen im Fingerbereich vermieden werden. Osteosynthese-Implantate im Fingerbereich kommen unter die Sehnen zu liegen. Bei einem Implantat mit grossem Querschnitt, kann die Sehne nicht mehr maximal gedehnt werden.

Die Dimensionen der sogenannten Mini-Implantate (Schrauben und Platten) liegen im Bereich von 0,8 mm - 2,0 mm. Durch diese Miniaturisierung sind Probleme im Bereich der Verpackung, Lagerung, sowie in der Handhabung beim Operationsvorgang entstanden. Die Handhabung im Operationssaal hat sich besonders im maxillofacialen Bereich, als schwierig erwiesen. Je nach Schwierigkeitsgrad der Fraktur oder Korrektur, werden bis zu 40 Knochenschrauben eingebracht. Diese Schrauben müssen einzeln von der OP-Schwester einem sogenannten Schraubenrechen entnommen, auf ihre Länge geprüft, auf einen Schraubenzieher gesetzt und dem Chirurgen übergeben werden. Dieser wiederum muss sie, durch die Osteosyntheseplatte, in das vorgebohrte Schraubenloch setzen. Bei der Schraubenübergahe und beim Versuch die Schraube einzudrehen, fällt diese oft vom Schraubenzieher, in die Wunde oder in die OP-Abdeckung. Der Versuch eine verlorene Schraube zu finden ist, bei deren Abmessungen (Durchmesser 0,8 mm x 4,0 mm), zu zeitaufwendig und würde die OP-Zeit verlängern. Da der Verlust von Schrauben durch die Handhabung im OP, bei der Verpackung und bei der Sterilisation recht häufig ist, entstehen dem Spital unnütze Kosten.

Ein weiteres Problem im Umgang mit Minischrauben wird nachfolgend beschrieben. Nachdem der Chirurg, die für die jeweilige Fraktur geeignete Osteosyntheseplatte ausgewählt hat, positioniert er die Platte über dem Frakturspalt. Danach bohrt er durch eines der Plattenlöcher die Kernbohrung für die Schraube (Durchmesser 0,5 - 1,5 mm). Nach dem Bohrvorgang übernimmt er von der OP-Schwester die benötigte Schraube und dreht diese durch die Platte in den Knochen. Das Problem dabei ist oft das Auffinden der Kernlochbohrung im Knochen, da die Knochenoberfläche mit Blut oder mit Weichteilen bedeckt ist und die Platte auf der glatten Knochenoberfläche rutschen kann.

Hier will die Erfindung Abhilfe schaffen. Der Erfindung liegt die Aufgabe zugrunde, eine Vorrichtung zur kontinuierlichen Darreichung und Applikation von osteosynthetischen Fixationselementen, insbesondere von Knochenschrauben zu schaffen. Eine weitere Aufgabe besteht darin eine Verpackung für solche osteosynthetische Fixationselemente zu schaffen, welche gleichzeitig zur Aufbewahrung im Operationssaal und als Dispensor für den Chirurgen dient.

Eine Vorrichtung zur Applikation von osteosynthetischen Fixationselementen ist aus US-A-3 171 408 bekannt.

Die Erfindung löst die gestellte Aufgabe mit einer Vorrichtung, welche die Merkmale des Anspruchs 1 aufweist, sowie einer Drehtrommel für diese Vorrichtung, welche die Merkmale des Anspruchs 9 aufweist.

Die durch die Erfindung erreichten Vorteile sind im wesentlichen darin zu sehen, dass dank der erfindungsgemässen Vorrichtung ein effizientes Werkzeug für den Chirurgen geschaffen wird, mit dem die Vorratshaltung für osteosynthetische Fixationselemente, insbesondere für Knochenschrauben, die intraoperative Darreichung und Applikation solcher Fixationselemente extrem erleichtert wird. Die erfindungsgemässe Vorrichtung dient neben der Darreichung der Fixationselemente gleichzeitig auch als Bohrlehre und Führung für den Schraubenzieher, mit dem die Fixationselemente, insbesondere Knochenschrauben eingedreht werden.

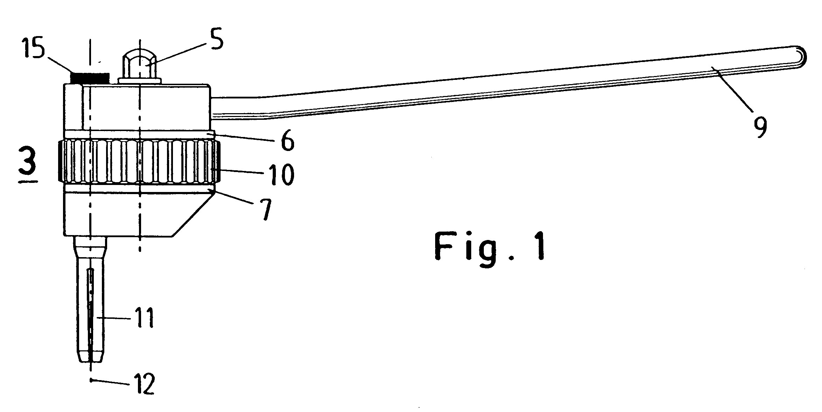

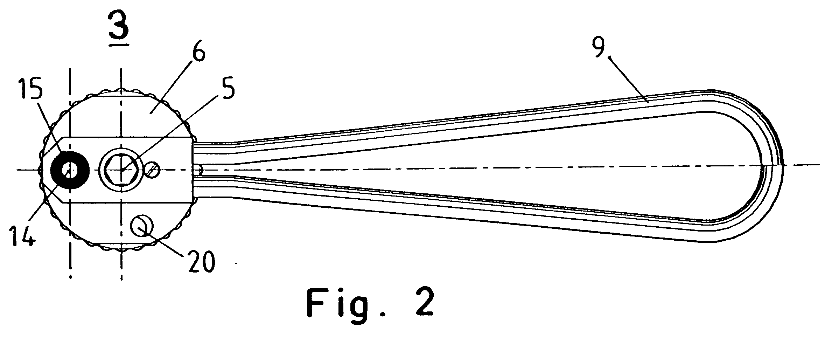

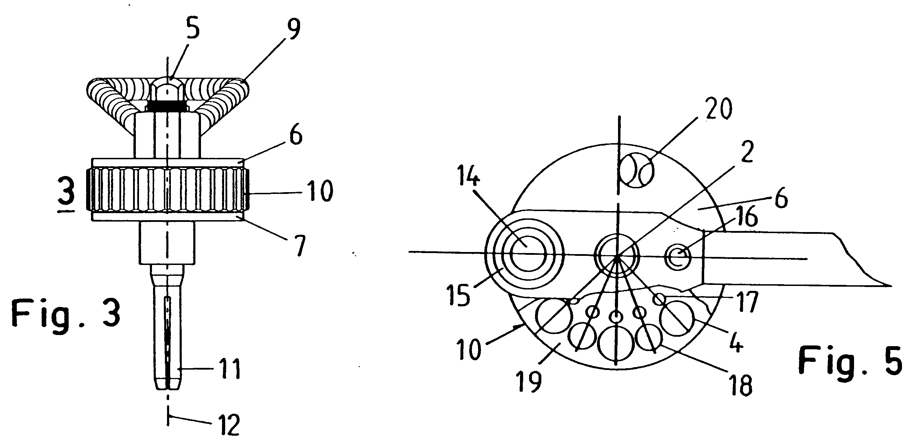

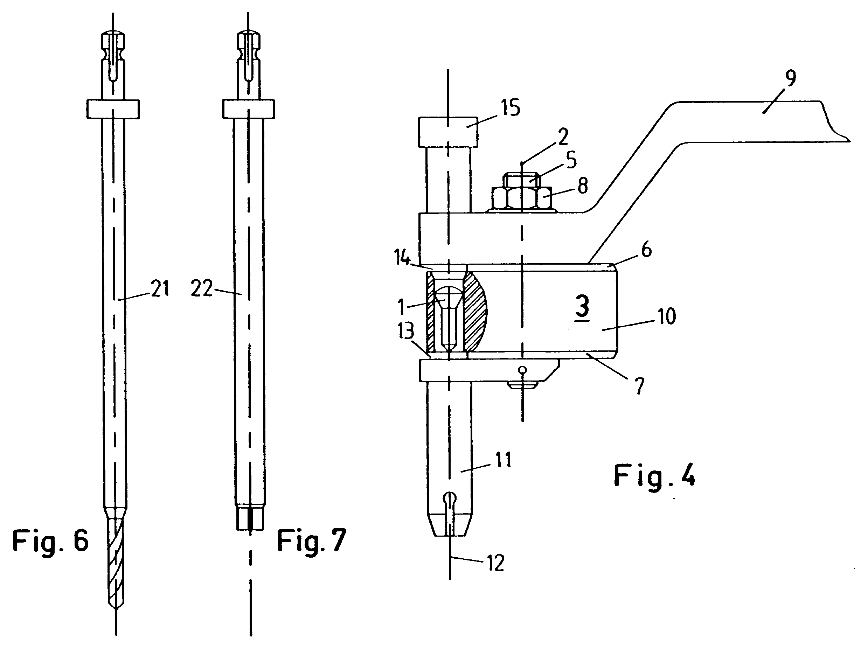

Ein Ausführungsbeispiel der Erfindung, welches zugleich das Funktionsprinzip erläutert, ist in der Zeichnung dargestellt und wird im folgenden näher beschrieben.
Fig. 1 stellt eine seitliche Ansicht der erfindungsgemässen Vorrichtung dar;
Fig. 2 stellt eine Aufsicht auf die erfindungsgemässe Vorrichtung dar;
Fig. 3 stellt eine Vorderansicht der erfindungsgemässen Vorrichtung dar.
Fig. 4 stellt einen Längsschnitt durch die erfindungsgemässe Vorrichtung dar;
Fig. 5 stellt eine partielle Aufsicht auf die erfindungsgemässe Vorrichtung dar;
Fig. 6 stellt eine seitliche Ansicht eines Knochenbohrers zur Verwendung mit der erfindungsgemässen Vorrichtung dar; und
Fig. 7 stellt eine seitliche Ansicht eines Schraubenziehers zur Verwendung mit der erfindungsgemässen Vorrichtung dar;
Die in den Fig. 1 - 5 dargestellte Vorrichtung gleicht in ihrem Aufbau einem Trommelrevolver. Die Trommeleinrichtung 3 besteht aus einem, eine Drehnabe 5 aufweisenden Trommelgehäuse 6,7 zur Aufnahme einer um die Achse 2 der Drehnabe 5 rotierbaren Drehtrommel 10, mit mehreren, peripher angeordneten und parallel zur Achse 2 verlaufenden, als Vorratskammern für Knochenschrauben 1 dienende durchgängige Bohrungen 4.
Das Trommelgehäuse 6,7 besteht seinerseits aus zwei durch die Drehnabe 5 verbundenen Tellern 6 und 7, wobei das Trommelgehäuse 6,7, beziehungswwiese die Teller 6 und 7, nicht auf der Drehnabe 5 rotiert werden können.
Der obere, fest mit einem Handgriff 9 verbundene Teller 6 ist mit einer Zugangsöffnung 14 zur Einführung eines in den Fig. 6 und 7 dargestellten Instrumentes 21 und 22 versehen, wobei das Zentrum der Zugangsöffnung 14 den gleichen Abstand zur Achse 2 aufweist wie die Zentren der peripher angeordneten und parallel zur Achse 2 verlaufenden, als Vorratskammern für die Knochenschrauben 1 dienenden durchgängigen Bohrungen 4.
Der untere, fest mit der Drehnabe 5 verbundenen Teller 7 ist mit einer Austrittsöffnung 13 für die Knochenschrauben 1 versehen, wobei das Zentrum der Austrittsöffnung 13 den gleichen Abstand zur Achse 2 aufweist wie die Zentren der Bohrungen 4.

Der untere Teller 7 des Trommelgehäuses 6,7 besitzt ferner eine Gewebeschutzhülse 11, durch welche die Kernlochbohrung und das Setzen der Knochenschrauben 1 vorgenommen werden kann. Der Abstand der Achse 12 der Gewebeschutzhülse 11 zur Achse 2, entspricht dem Abstand der Schrauben- und Bohrlöcher 4, 18 in der Drehtrommel 10 zu ihrer Zentrumsachse 2. Der obere Teller 6 besitzt neben dem Handgriff 9 eine Bohrbüchse 15, die mit den Bohrungen 4 und 18 und der Achse 12 der Gewebeschutzhülse 11 übereinstimmt. Zudem besitzt der obere Teller einen gefederten Bolzen oder Kugel, die in die Indexierungen 17 der Drehtrommel 10 eingreifen kann. Dadurch können die Bohrungen 4 und 18 der Drehtrommel 10 mit der Gewebeschutzhülse 11 und der Bohrbüchse 15 einfach ausgerichtet werden.

Zur Montage der erfindungsgemässen Vorrichtung wird die fest mit dem unteren Teller 7 verbundene Drehnabe 5 durch die Hohlnabe der Drehtrommel 10 und eine (zeichnerisch nicht dargestellte) Öffnung im oberen Teller 6 hindurchgeführt und mit der Mutter 8 in einem bestimmten, die Rotierbarkeit der zwischen den beiden Tellern 6,7 drehbar gelagerten Drehtrommel 10 gewährleistenden, Abstand fixiert.

Die eigentliche Verpackung der Knochenschrauben 1, oder schraubenähnlichen Fixationselemente, ist somit die Drehtrommel 10 selbst, welche zirkulär angeordnete Bohrungen 4 und 18 besitzt. Dabei gibt es zwei verschiedene Bohrdurchmesser die sich untereinander abwechseln. Die grösseren Bohrungen 4 dienen zur Aufnahme der Knochenschrauben 1, die kleineren Bohrungen 18 zur Führung des Bohrers 21, für die Kernlochbohrung im Knochen, sowie des Schraubenziehers 22 zur Befestigung der Knochenschraube 1 im Knochen. Eine kreisförmig angebrachte Indexierung 17 auf der Trommeloberseite 19, erlaubt eine genau definierte Positionierung der gewünschten Bohrung 4 oder 18 innerhalb des Trommelgehäuses 6,7.

Die Drehtrommel 10 ist einfach auswechselbar und dient gleichzeitig als Verpackung für die Knochenschrauben 1. Je nach Bedarf kann die Drehtrommel in einer sterilen Verpackung angeboten werden, oder sie kann erst im Spital sterilisiert werden. Statt die Drehtrommel 10 nach ihrer Erschöpfung mit einer neuen vollen Drehtrommel 10 auszutauschen kann sie auch über eine zusätzliche, im oberen Teller 6 angebrachte Einfüllbohrung 20 mit Knochenschrauben 1 nachgefüllt werden. Das Zentrum der Einfüllbohrung 20 muss konstruktionsbedingt den gleichen Abstand zur Achse 2 aufweisen wie die Zentren der Bohrungen 4.

Im folgenden wird die intraoperative Handhabung der erfindungsgemässen Vorrichtung beschrieben:
Die mit Knochenschrauben 1 gefüllte Drehtrommel 10 wird in das Trommelgehäuse 6,7 geschoben, ohne die einzelnen Knochenschrauben 1 handhaben zu müssen. Der Chirurg benutzt nun die erfindungsgemässe Vorrichtung vorerst als Bohrbüchse, um die Kernlochbohrung für die zu setzende Knochenschrauben 1 in den Knochen zu bohren. Dabei wird der Bohrer 21 durch die Bohrbüchse 15 des oberen Tellers 6, durch eine der kleineren Bohrungen 18 der Drehtrommel 10 und durch die Gewebeschutzhülse 11 exakt geführt. Die Länge der einzelnen Führungselemente 15,11 sind mit der Länge der Bohrung abgestimmt. Dadurch kann die Bohrtiefe im Knochen, auf die Schraubenlänge angepasst werden. Die Spitze der Gewebeschutzhülse 11 ist auf die Schraubenlöcher in der Knochenplatte angepasst, wodurch die Knochenplatte auf den Knochen gedrückt wird und dadurch nicht mehr abrutschen kann. Der Chirurg führt nun die Kernlochbohrung im Knochen durch. Nach Entfernung des Bohrers 21, dreht der Chirurg die Drehtrommel 10 mittels ihrer peripheren Randrierung um eine Indexposition 17 weiter. Dadurch gelangt eine der, sich in der Drehtrommel 10 befindlichen Knochenschrauben 10, in den durch die Elemente 13 und 11 gebildeten Arbeitskanal. Je nach Beschaffenheit der Bohrung 4 in der sich die Knochenschraube 1 befindet, fällt die Knochenschraube 1 durch die Gewebeschutzhülse 11 auf die Knochenoberfläche, oder sie muss mit einem Schraubenzieher 22, durch die Gewebeschutzhülse 11 auf die Knochenoberfläche geschoben werden. Der Durchmesser des Schraubenziehers 22 ist auf den Kopfdurchmesser der Knochenschraube 1 angepasst, so dass er auf seiner ganzen Länge geführt ist und gezwungenermassen in den Antrieb der Knochenschraube 1 eingreift (Kreuzschlitz, Inbus usw.). Da die erfindungsgemässe Vorrichtung während des ganzen Arbeitsvorganges nicht entfernt werden muss, kann die Knochenschraube 1, ohne Aufsuchen der Pilotbohrung im Knochen eingedreht werden.
Nachdem die Knochenschraube 1 vollständig eingedreht ist, wird der Schraubenzieher 22 entfernt und die Drehtrommel 10 um eine Indexposition 17 weiter gedreht. Dadurch befindet sich wiederum eine kleinere Bohrung 18 der Drehtrommel 10 im Arbeitskanal 13,11, wodurch die nächste Kernlochbohrung vorgenommen werden kann. Der gesamte Vorgang wird wiederholt, bis alle Schraubenlöcher der Platte mit Knochenschrauben 1 belegt sind.

## Patentansprüche

1. Vorrichtung zur kontinuierlichen Darreichung von osteosynthetischen Fixationselementen (1), mit einer um ihre Achse (2) drehbaren Trommeleinrichtung (3), dadurch gekennzeichnet dass
A) die Trommeleinrichtung (3) eine Drehnabe (5) umfasst, zur Aufnahme einer rotierbaren Drehtrommel (10) mit mehreren, peripher angeordneten und parallel zur Achse (2) verlaufenden, als Vorratskammern für osteosynthetische Fixationselemente (1) dienenden, durchgängige Bohrungen (4); und
B) die Drehtrommel (10) auf einem, fest mit der Drehnabe (5) verbundenen, unteren Teller (7) mit einer Austrittsöffnung (13), für die in den Bohrungen (4) gelagerten, osteosynthetischen Fixationselemente (1), drehbar lagerbar ist, wobei das Zentrum der Austrittsöffnung (13) den gleichen Abstand zur Achse (2) aufweist wie die Zentren der Bohrungen (4).

2. Vorrichtung nach Anspruch 1, dadurch gekennzeichnet, dass sie zusätzlich einen oberen, vorzugsweise fest mit einem Handgriff (9) verbundenen, die Drehtrommel (10) bedeckenden Teller (6) mit einer Zugangsöffnung (14) für die in den Bohrungen (4) gelagerten, osteosynthetischen Fixationselemente (1) aufweist, wobei das Zentrum der Zugangsöffnung (14) den gleichen Abstand zur Achse (2) aufweist wie die Zentren der Bohrungen (4).

3. Vorrichtung nach Anspruch 2, dadurch gekennzeichnet, dass Befestigungsmittel (8) zur gegenseitigen Fixierung der beiden Teller (6,7) in einem bestimmten, die Rotierbarkeit der zwischen den beiden Tellern (6,7) aufzunehmenden Drehtrommel (10) gewährleistenden, Abstand umfasst.

4. Vorrichtung nach Anspruch 3, dadurch gekennzeichnet, dass der untere Teller (7) eine parallel zur Achse (2) nach unten verlaufende, an die Austrittsöffnung (13) anschliessende Gewebeschutzhülse (11) aufweist.

5. Vorrichtung nach Anspruch 3 oder 4, dadurch gekennzeichnet, dass der obere Teller (6) eine parallel zur Achse (2) nach oben verlaufende, an die Zugangsöffnung (14) anschliessende Bohrbüchse (15) aufweist.

6. Vorrichtung nach einem der Ansprüche 3 - 5, dadurch gekennzeichnet, dass der obere Teller (6) eine Einrastvorrichtung (16), vorzugsweise einen gefederten Bolzen oder Kugel, zur schrittweisen, lösbaren Blockierung und Positionierung der mit entsprechenden Positionier-Ansenkungen (17) versehenen Drehtrommel (10) aufweist.

7. Vorrichtung nach einem der Ansprüche 3 - 6, dadurch gekennzeichnet, dass der obere Teller (6) eine zusätzliche, zur Nachfüllung der Vorrichtung mit osteosynthetischen Fixationselementen (1) dienende Einfüllbohrung (20) aufweist, wobei das Zentrum der Einfüllbohrung (20) den gleichen Abstand zur Achse (2) aufweist wie die Bohrungen (4), wobei die Einfüllbohrung (20) bei Eingriff der Einrastvorrichtung (16) in die Positionier-Ansenkungen (17) zwischen den Zentren der Bohrungen (4) und (18) angeordnet ist.

8. Vorrichtung nach einem der Ansprüche 1 - 7, dadurch gekennzeichnet, dass die Bohrungen (4) zur Aufnahme von Knochenschrauben ausgebildet sind.

9. Drehtrommel für eine Vorrichtung gemäss einem der Ansprüche 1 - 8, dadurch gekennzeichnet, dass zwischen den peripher angeordneten Bohrungen (4) zusätzliche als Führung für Instrumente (21,22) dienende Bohrlöcher (18) vorgesehen sind.

10. Drehtrommel nach Anspruch 9, dadurch gekennzeichnet, dass sie auf ihrer Oberseite (19) radial zu jeder Bohrung (4) und jedem Bohrloch (18) mit je einer Positionier-Ansenkungen (17) versehen ist.

11. Drehtrommel nach Anspruch 9 oder 10, dadurch gekennzeichnet, dass sie als, vorzugsweise sterile, verpackungseinheit für die osteosynthetischen Fixationselemente (1) ausgebildet ist.

## Claims

1. Device for the continuous presentation of osteosynthesis fixation elements (1) with a drum device (3) that can be rotated around its axis (2), characterized in that
A) the drum device (3) includes a rotary hub (5) for receiving a rotatable drum (10) with several through holes (4) arranged peripherally and parallel to the axis (2), serving as storage chambers for osteosynthesis fixation elements (1); and
B) the rotatable drum (10) is carried rotatably by a lower plate (7) solidly connected to the rotary hub (5), said lower plate (7) having an outlet opening (13) for the osteosynthesis fixation elements (1) stored in the through holes (4), whereby the center of the outlet opening (13) has the same distance from the axis (2) as the center of the through holes (4).

2. Device according to claim 1, characterized in that it further comprises an upper plate (6) covering the rotatable drum (10) and preferably connected solidly to a handle (9), with an access opening (14) for the osteosynthesis fixation elements (1) stored in the through holes (4), whereby the center of the access opening (14) has the same distance from the axis (2) as the through holes (4).

3. Device according to claim 2, characterized in that it includes fastening means (8) for the mutual fixation of the two plates (6, 7) at a certain distance guaranteeing the turnability of the rotary drum (10) to be placed between the two plates (6,7).

4. Device according to claim 3, characterized in that the lower plate (7) has a tissue-protection sleeve (11) connected to the outlet opening (13), running downwards parallel to the axis (2).

5. Device according to claim 3 or 4, characterized in that the upper plate (6) has a drill bush (15) connected to the access opening (14), running upwards parallel to the axis (2).

6. Device according to one of the claims 3 - 5, characterized in that the upper plate (6) has an engaging device (16), preferably a spring-loaded bolt or ball, for the step-by-step, releasable locking and positioning of the rotatable drum (10) provided with corresponding positioning countersinks (17).

7. Device according to one of the claims 3 - 6, characterized in that the upper plate (6) has an additional filling drill hole (20) serving to refill the device with osteosynthesis fixation elements (1), whereby the center of the filling drill hole (20) is arranged at the same distance from the axis (2) as the through holes (4), whereby upon engagement of the engaging device (16) with the positioning countersinks (17) the filling drill hole (20) is arranged between the centres of the through holes (4) and (18).

8. Device according to one of the claims 1 - 7, characterized in that the through holes (4) are formed to receive bone screws.

9. Rotary drum for a device according to one of the claims 1 - 8, characterized in that additional through holes (18) serving for the guidance of instruments (21, 22) are provided between the peripherally arranged through holes (4).

10. Rotary drum according to claim 9, characterized in that it is provided, on its upper side (19), with positioning countersinks (17) placed radially to each through hole (4) and each through hole (18).

11. Rotary drum according to claim 9 or 10, characterized in that it is formed as a, preferably sterile, packaging unit for the osteosynthesis fixation elements (1).

## Revendications

1. Dispositif pour la présentation en continu d'éléments (1) de fixation ostéosynthétique, comportant un dispositif à barillet (3) pouvant tourner autour de son axe (2), caractérisé en ce que
A) le dispositif à barillet (3) comprend un moyeu de rotation (5) pour la réception d'un barillet rotatif (10) comportant plusieurs alésages traversants (4) disposés à la périphérie, qui s'étendent parallèlement à l'axe (2) et servent de chambres de réserve pour des éléments de fixation ostéosynthétique (1); et
B) le barillet rotatif (10) est monté à rotation sur un plateau inférieur (7) solidaire du moyeu de rotation (5) et comportant une ouverture de passage (13) pour les éléments de fixation ostéosynthétique (1) maintenus dans les alésages (4), le centre de l'ouverture de sortie (13) présentant par rapport à l'axe (2) le même écart que les centres des alésages (4).

2. Dispositif selon la revendication 1, caractérisé en ce qu'il présente en outre un plateau supérieur (6) recouvrant le barillet rotatif (10), de préférence solidaire d'une poignée (9), comportant une ouverture d'accès (14) pour les éléments de fixation ostéosynthétique (1) maintenus dans les alésages (4), le centre de l'ouverture d'accès (14) présentant par rapport à l'axe (2) le même écart que les centres des alésages (4).

3. Dispositif selon la revendication 2, caractérisé en ce qu'il comporte des moyens de fixation (8) pour la fixation mutuelle des deux plateaux (6, 7) à une distance définie assurant la possibilité de rotation du barillet rotatif (10) repris entre les deux plateaux (6, 7).

4. Dispositif selon la revendication 3, caractérisé en ce que le plateau inférieur (7) présente un manchon (11) de protection de tissu, s'étendant vers le bas parallèlement à l'axe (2) et se raccordant à l'ouverture de sortie (13).

5. Dispositif selon la revendication 3 ou 4, caractérisé en ce que le plateau supérieur (6) présente une douille de forage (15) s'étendant vers le haut parallèlement à l'axe (2) et se raccordant à l'ouverture d'accès (14).

6. Dispositif selon l'une quelconque des revendications 3 à 5, caractérisé en ce que le plateau supérieur (6) présente un dispositif d'accrochage élastique (16), de préférence un bouton ou une sphère à ressort, pour le blocage et le positionnement pas-à-pas et libérable du barillet rotatif (10) doté de chanfreins de positionnement correspondants (17).

7. Dispositif selon l'une quelconque des revendications 3 à 6, caractérisé en ce que le plateau supérieur (6) présente un alésage de remplissage supplémentaire (20), servant à remplir le dispositif en éléments de fixation ostéosynthétique (1), le centre de l'alésage de remplissage (20) présentant par rapport à l'axe (2) le même écart que les alésages (4), l'alésage de remplissage (20) étant disposé entre les centres des alésages (4) et (18) lorsque le dispositif d'accrochage élastique (16) est accroché dans les chanfreins de positionnement (17).

8. Dispositif selon l'une quelconque des revendications 1 à 7, caractérisé en ce que les alésages (4) sont configurés pour la réception de vis à os.

9. Barillet rotatif pour un dispositif selon l'une quelconque des revendications 1 à 8, caractérisé en ce qu'entre les alésages (4) disposés à la périphérie, des trous de forage supplémentaires (18) sont prévus pour servir de guide pour des instruments (21, 22).

10. Barillet rotatif selon la revendication 9, caractérisé en ce qu'il est doté sur sa face supérieure (19), radialement par rapport à chaque alésage (4) et à chaque trou de forage (18), chaque fois avec un chanfrein de positionnement (17).

11. Barillet rotatif selon la revendication 9 ou 10, caractérisé en ce qu'il est configuré comme unité d'emballage, de préférence stérile, pour les éléments de fixation ostéosynthétique (1).
